# EUROPEAN PATENT SPECIFICATION

(11) **EP 3 581 240 B1**
(45) Date of publication and mention of the grant of the patent: **01.12.2021**
(21) Application number: 18751638.0
(22) Date of filing: 12.02.2018
(51) Int. Cl.: A61N 5/00, A61B 6/04, A61G 13/12

(54) **IMMOBILISATION DEVICE FOR RADIOTHERAPY**
IMMOBILISIERUNGSVORRICHTUNG FÜR DIE STRAHLENTHERAPIE
DISPOSITIF D'IMMOBILISATION POUR RADIOTHÉRAPIE

(30) Priority: 10.02.2017 ES 201730166
(43) Date of publication of application: 18.12.2019
(73) Proprietor: Servicio Andaluz de Salud, 41071 Sevilla (ES)
(72) Inventor: VELÁZQUEZ MIRANDA, Santiago, 41013 Sevilla (ES); ORTIZ SEIDEL, Mónica, 41071 Sevilla (ES)
(74) Representative: Hoffmann Eitle
(86) International application number: PCT/ES2018/070099
(87) International publication number: WO 2018/146367

(56) References cited:
- EP-A1- 1 897 497
- CN-A- 105 327 458
- CN-U- 204 563 326
- CN-U- 204 563 326
- CN-U- 204 563 327
- CN-U- 204 563 327
- FR-A1- 2 765 487
- JP-A- H1 033 601
- JP-A- H1 033 601
- JP-A- H01 153 142
- JP-A- H01 153 142
- KR-A- 20130 090 069
- KR-A- 20130 090 069
- US-A1- 2003 164 459
- US-A1- 2005 085 722
- US-A1- 2007 074 347
- US-A1- 2009 308 400
- US-A1- 2009 308 400
- US-A1- 2011 278 477
- US-A1- 2016 228 326

## Description

### OBJECT OF THE INVENTION

The present invention generally belongs to the field of medicine, and more specifically to the treatment of cancer by means of radiotherapy.

The object of the present invention is a novel device designed for immobilising a patient during radiotherapy treatment.

### BACKGROUND OF THE INVENTION

Radiotherapy treatment consists of applying high radiation doses to a cancer-affected organ or tissue for the purpose of destroying cancer cells. Body movements, such as those due to the patient's postural changes or breathing itself, for example, make it difficult to concentrate the radiation on the target volume to be irradiated. Various types of immobilisation devices are available today to solve this problem, the purpose of which is to immobilise, in the best way possible, the area of the patient on which radiotherapy treatment will be performed. These immobilisation devices include, among others, those designed for the treatment of the chest area or breasts of a patient in a supine position.

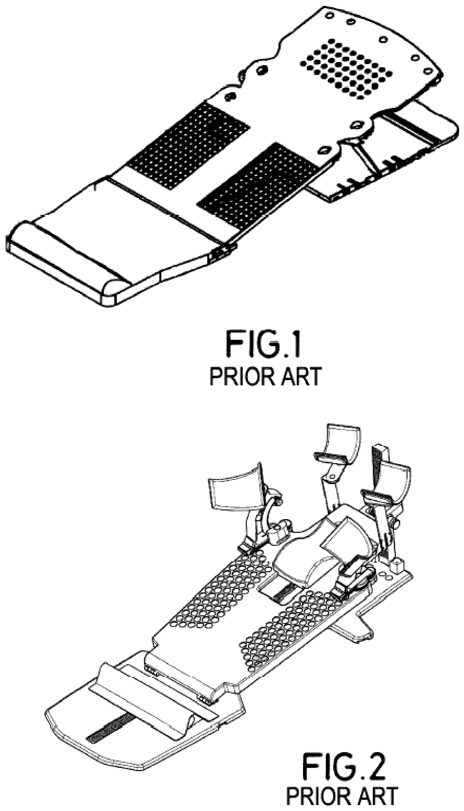

By way of example, Figures 1 and 2 show respective examples of devices of this type known today. Specifically, Figure 1 corresponds to an immobilisation device described in document US2005085722 and Figure 2 corresponds to an immobilisation device described in document KR20130090069. Numerous commercial products of this type, such as for example, C-Qual Breast Board by Civco or MammoRX Breast Board by Orfit, among many others, are also available today. As can be seen, these devices known today are fundamentally formed by an inclined plate, intended for supporting the back of the patient, which is connected to a horizontal platform intended for supporting the buttocks of the patient. The inclined plate has a variable angle of inclination and is provided at its upper end with arm restraining elements and a headrest. The arm restraining elements are used to keep the arms of the patient above their head, away from the treatment area, whereas the headrest allows the patient to rest their head comfortably during treatment. The horizontal platform has a flange or step intended for the patient to rest their buttocks and thereby prevent them from sliding down.

Devices of this type sufficiently immobilises the patient for conventional radiotherapy treatments which are based on the application of very low radiation doses split into many radiotherapy sessions, given that in these cases it is possible for part of the radiation to impact outside of the target volume. However, a new technique referred to as stereotactic ablative radiotherapy (SABR), also known as stereotactic body radiation therapy (SBRT), is being developed today. This new technique fundamentally consists of applying very high radiation doses split into very few radiotherapy sessions, ideally a single radiotherapy session. In this case, given the high radiation intensity, the amount of radiation received by tissues surrounding the target volume must be minimized. In this context, achieving an improved patient immobilisation with respect to that provided by devices known today is crucial.

There is therefore an unmet need in this field of the art for immobilisation devices capable of immobilising a patient in a precise and repeatable manner.

### DESCRIPTION OF THE INVENTION

The object of the invention is a stereotactic immobilisation device which allows increasing reproducibility of breast tumour localization with respect to radiation treatment machines (linear electron accelerators) and imaging machines (CT scan, PET, and MR). In other words, one and the same patient must move between different machines and the tumour must have the same coordinates for all of said machines. It fundamentally consists of a rotatable pelvic supporting platform that can be connected to a truncated inclined plane for supporting the back and an integral arm for restraining the arms.

In this document, *"longitudinal direction of the immobiliser"* refers to, unless explicitly stated otherwise, a direction parallel to the long side of the inclined plate, which coincides with the craniocaudal direction of the patient when they are resting on the inclined plate.

In this document, *"transverse direction of the immobiliser"* refers to, unless explicitly stated otherwise, a direction perpendicular to the preceding longitudinal direction, which coincides with the left-right side direction of the patient when they are resting on the inclined plate.

In this document, *"sides"* of the lower platform or of the inclined plate refer to the edges along the longest side of the immobilisation device. In other words, they are the edges parallel to the longitudinal direction of the immobiliser.

In this document, the term *"reproducibility"* refers to the capacity to precisely reproduce or replicate the position of a patient to assure tumour localization during a given radiotherapy treatment, both intra-treatment, i.e., during specific treatment of one and the same patient, and inter-treatment, i.e., between different treatments performed in different radiation treatment machines (linear electron accelerators) and imaging machines (CT scan, PET, and MR).

In this document, the term *"stereotactic device"* is that which allows visually linking the patient's external anatomy with coordinates marked in the device and with lasers that orthogonally trace the reference planes of the different machines involved in the radiotherapy process, and once the radiological image is acquired those coordinates remain visible therein, thereby allowing the external configuration of the equipment to be linked with the internal organs and tumours of the patient.

In this document, *"patient'* is used at all times to refer to the user of the immobilisation device. However, it must be interpreted that this term covers both male and female patients, particularly taking into account that it is mainly intended for use in SBRT breast cancer treatments.

The invention is as defined in the appended claims. The present disclosure describes an immobilisation device for radiotherapy, fundamentally comprising:

### a) An inclined plate

It is a plate configured for supporting the back of the patient which is inclined in a longitudinal direction and has a lower end and an upper end. It is usually a flat plate having an essentially rectangular shape inclined in the direction of its longest side, such that the patient can lean back and rest both their back and their head thereon. As mentioned herein above, the longitudinal direction coincides with the craniocaudal direction of the patient when they are immobilised on the device of the invention. The craniocaudal inclination allows the breast to be positioned by gravity itself, thereby aiding in the reproducibility of the position of the breast.

The inclined plate can furthermore have an area intended for supporting the patient's head. This area can be provided with, for example, a headrest or the like and means which allow moving the headrest up or down according to the longitudinal direction of the inclined plate depending on the patient's height.

### b) A lower platform

The lower platform is intended for supporting the buttocks of the patient and configured for being placed adjacent to the lower end of the inclined plate. The patient can thereby rest their buttocks on the lower platform and lean back on the inclined plate, being relatively immobilised and ready to receive specific radiotherapy treatments.

In preferred embodiments of the invention, the lower platform can be coupled to the lower end of the inclined plate. In this case, the coupling between the lower platform and the inclined plate can be carried out in any way known in the art, for example by means of screw connections, press fits, tongue and groove joints, dovetail joints, etc. The purpose is to prevent the lower platform from being able to slide away from the lower end of the inclined plate as a result of the stresses produced when a patient rests on the immobilisation device. More preferably, the coupling between the lower platform and the inclined plate can have a variable length, such that the distance between both elements can be modified. This will allow moving the lower platform away from the lower end of the inclined plate in the case of taller patients, and in a contrary manner moving the lower platform closer to the lower end of the inclined plate in the case of shorter patients. For example, the coupling can be made with help of a pair of toothed rods arranged in one of the two elements which fit, by way of a ratchet wrench, into corresponding openings arranged in the other element.

Alternatively, the lower platform can constitute a separate element that cannot be coupled to the lower end of the inclined plate. It can therefore be arranged at a distance from said lower end of the inclined plate which is required for each patient. Furthermore, in this case the lower platform can have fixing means for fixing to the stretcher of the apparatus on which it is arranged.

These elements are equivalent to common elements in the immobilisation devices known today. However, the device of the present invention is characterised in that the lower platform is rotatable to make it easier to place the patient on the device. This rotatable platform can have a circular-shaped rotatable portion preferably with a radius between 20 cm and 30 cm, more preferably about 25 cm.

In fact, the devices known today have a fixed lower platform which the patient must get onto in order to be placed on the device. It should be noted that the described immobilisation device will normally be placed, during use, on the stretcher of a linear accelerator. Therefore, for placement on a conventional immobilisation device, the patient must first sit on the lower platform according to a transverse orientation, i.e., with their legs sticking out over the side edge of the lower platform (and therefore also the side edge of the stretcher of the linear accelerator). The patient must then lift their legs and, supporting themselves on their buttocks and with the help of their hand, perform a turn which places them aligned with the longitudinal direction of the device. Their legs are supported at this point on the lower part of the stretcher of the linear accelerator on which the immobilisation device is placed. Lastly, the patient must move, *"dragging themselves"* with the help of their hands, until their buttocks are placed on the flange or step arranged in the lower platform. Finally, the patient leans back to rest their back on the inclined plate. This method entails a drawback in many ways. First, some patients do not have sufficient mobility to carry out these steps with ease, for example, due to excess weight, old age, extreme weakness, etc. This makes it necessary for medical personnel to help the patient, with the subsequent effort and loss of time associated therewith. Moreover, the need for the patient to perform various movements supported on their buttocks and hands, *"dragging themselves"* to the suitable position, with or without help, entails serious drawbacks in terms of the reproducibility of the final position. It can be said that the patient is placed on the device in a position different from the preceding one every single time. It should be noted that, in the context of the reproducibility of patient positioning results, it is known that the greater the need for patient collaboration, the worse the results will be.

The arrangement of the rotatable platform solves these problems, since the patient can sit on the lower platform sideways in a transverse direction, with their legs sticking out over the side edge of the lower platform, and the platform itself will carry out a 90° turn to place the patient according to the longitudinal direction. Therefore, the only action the patient has to do is fundamentally to sit on a specific position on the lower platform. Given that the stretcher of a linear accelerator on which the immobilisation device is normally arranged can be lowered, it is an action that is within reach of almost any patient. Once the patient is sitting in the desired position, the medical personnel can raise the patient's legs and turn the rotatable platform 90° until aligning the craniocaudal direction of the patient with the longitudinal direction of the device. Therefore, the patient only has to lean back to rest on the inclined plate without having to do anything more, without altering their position with the loss of supports entailed upon lifting their legs, given that the pelvis is already fitted in the platform. The present device therefore allows patients with reduced mobility to be placed on the device much more easily and quickly. Furthermore, it improves the reproducibility of the final position as it is much easier for the patient to sit directly on a specific position than to drag themselves with the help of their hands to said position on a horizontal surface.

An orientation of the rotatable lower plate designed so that the patient sits according to the transverse direction at the start of the immobilisation method will herein be referred to as *"initial orientation".* The orientation of the rotatable lower plate which, after a 90° turn in relation to the initial orientation, allows aligning the craniocaudal direction of the patient seated thereon with the longitudinal direction will be referred to as *"usage orientation".*

Additionally, the inventor of the present application has also discovered that the transverse flange or step intended for supporting the buttocks of the patient on the known immobilisation devices does not constitute a suitable immobilisation element. In fact, the area of the buttocks of the patient does not constitute a firm supporting area as it has a large amount of soft tissues, such as fat and muscle, which may shift in position according to the posture the patient adopts. As a result, a patient can adopt numerous positions despite having their buttocks firmly supported on a transverse flange or step like the one used in the devices of the prior art, depending on the particular configuration the fat and muscle of their buttocks adopt.

To solve this problem, the present invention is further characterized in that the lower platform comprises a vertically projecting protuberance which is transversely centred in relation to the inclined plate when it is in the usage orientation. This protuberance is configured such that it can be introduced between the legs of the patient for them to rest their perineum, thereby preventing the sliding of the patient and assuring their position in a more precise manner than the conventional flange. In this context, the expression *"vertically projecting"* means that the protuberance is raised above the surface of the lower platform, although it is not essential for it to be a strictly vertical element in the sense of forming 90° in relation to said surface. In terms of its position, the protuberance must be centred transversely in relation to the side edges of the inclined plate when the lower rotatable platform is connected to said inclined plate and adopts the usage orientation. However, it is important to highlight that the protuberance does not have to be arranged in the centre of rotation of the lower platform. For example, the protuberance can be located at a distance between 6 cm and 12 cm from the centre of rotation, more preferably at about 9 cm. This protuberance allows immobilising the patient against movements in the longitudinal direction of the immobilisation device in a much more precise manner, since the inner thigh is an area with much less fat than the buttocks. The support is firmer, and therefore the resulting position much more reproducible. In principle, the protuberance can adopt any shape suitable to be comfortably supported on the inner thigh of the patient, although it preferably has a cross-section with smooth curves that are devoid of sharp edges to prevent injuring the patient, for example with a circular, elliptical, or oval shape.

According to another preferred embodiment of the device of the present invention, the lower platform further comprises in lateral areas according to the usage orientation, on both sides of the protuberance for supporting the perineum, a pair of vertically projecting plates. These plates are configured for compressing the hips and securing the position of the patient even further, and thereby preventing lateral movements of the pelvis and unwanted sliding. These plates will adopt symmetrical positions in relation to a longitudinal vertical plane of the immobiliser when the rotatable platform is in its usage orientation. The vertical plates limit the lateral movements of the hips of the patient, even further improving the reproducibility of the immobilisation, and furthermore providing a restraining site for improved seating. It should be noted that it is not essential for these plates to project strictly in the vertical direction, where a small angle can be formed in relation to the lower platform. The distance between the plates will be selected taking into account anatomical parameters of the patients' build, although it can preferably be between 35 cm and 50 cm, more preferably about 40 cm. The plates preferably have a length between 10 and 15 cm, more preferably about 12 cm, and their height can be between 10 cm and 20 cm, more preferably about 15 cm.

As a result of these plates, once the patient sits on the rotatable platform, they are completely *"fitted'* and have no possibility of lateral movement. In combination with the central protuberance, complete immobilisation of the pelvis of the patient is achieved, which translates into greater reproducibility of the position of their entire upper trunk.

The inventor of the present application has verified that another reason for the poor results obtained with current immobilisation devices is related to the movements of the patient's trunk, since these movements are not limited in any way because the inclined plates used in the prior art are completely flat. To solve this problem, in another preferred embodiment of the invention the inclined plate is formed by two V-shaped side sub-plates which are additionally inclined in the transverse direction of the device for the purpose of making it difficult for the patient to turn. In other words, the plate is not only inclined in the longitudinal direction, but is furthermore formed by two flat side portions which are additionally inclined in a transverse direction, i.e., towards the central longitudinal line of the inclined plate itself, the inclined plate thereby adopting an essentially V shape. This thereby makes it extremely difficult for the patient to turn toward either side. For example, the transverse angle of inclination can be between about 10 and 20°.

According to another more preferred embodiment of the invention, the inclined plate further comprises two side contention walls projecting essentially in a vertical manner for closely housing the patient and making their movement even more difficult. The patient's trunk is thereby completely constrained between the two side walls, even further improving the reproducibility of the immobilisation. The distance between these two walls will be selected according to standard patient distances, although it will preferably be between 35 cm and 50 cm, more preferably about 42 cm. The height of the contention walls will preferably be between 7 and 15 cm, more preferably about 9 cm.

In another preferred embodiment of the invention, the inclined plate comprises a central longitudinal cavity to make it easier to position the spinous process of the patient. This cavity prevents discomfort when the patient rests on the inclined plate, furthermore making it easier for their back to be suitably centred on the inclined plate and improving the immobilisation and reproducibility of the position of the patient. For example, if the plate is formed by two sub-plates additionally inclined in a transverse direction as described above, the central longitudinal cavity can constitute the separation between both sub-plates. In other words, the longitudinal cavity would be arranged on the vertex of a V shape formed by the cross-section of both sub-plates. The width of the central longitudinal cavity is preferably between 4 cm and 8 cm, more preferably about 6 cm.

In terms of the inclination of the inclined plate, the immobilisation devices known up until now have means for modifying inclination according to each particular case. However, the inventor of the present application has discovered that this constitutes another reason for the lack of reproducibility in immobilisation and that it is not actually essential for the inclination to be modifiable. For that reason, according to another preferred embodiment of the present invention the inclined plate is configured such that its angle of inclination is fixed and cannot be modified. This angle can preferably be between 0° and 45°, more preferably between 5° and 15°, and even more preferably it can be about 10°.

The inventor of the present application has also discovered that another reason for the lack of reproducibility in patient immobilisation is related to the discomfort of the position in which the devices of the prior art immobilise the arms. In fact, in the known devices arms are immobilised in a position in which the patient's hands are in a position located longitudinally above their head. This position is very uncomfortable, and as a result the patients move more than what would be desirable.

To solve this problem, according to still a more preferred embodiment of the invention the inclined plate comprises at its upper end arm restraining means comprising a restraining element. The restraining element is movable between a retracted position located longitudinally above a supporting position for supporting the patient's head, and an extended position located longitudinally at the height of the supporting position for supporting the patient's head. Furthermore, in the extended position the lower end of the restraining element is perpendicularly separated from the inclined plate by a distance that is enough for accommodating the patient's head such that it is supported on said supporting position for supporting the patient's head. For example, the distance between the plate and the lower end of the restraining element when said restraining element is in the extended position is preferably between 20 cm and 30 cm, more preferably between 24 and 28 cm, and even more preferably about 26 cm.

As a result of this configuration, the patient first rests their head in a head supporting position with the restraining element in the retracted position. In this position, the restraining element does not constitute an obstacle for the placement of the patient's head on the supporting position because it is arranged in a position longitudinally above said supporting position for supporting the patient's head. As mentioned herein above, the head supporting position can include a headrest. Furthermore, the headrest can be sliding in the longitudinal direction along the inclined plate, such that it can be placed in the position that best suits the height of each patient. Once the patient's head has been positioned on the supporting position of the inclined plate, the arm restraining element is moved from the retracted position to the extended position. In the extended position, the arm restraining element is arranged at the height of the patient's head, for example at the height of their eyes or forehead, and at a specific distance from same according to a direction perpendicular to the inclined plate. The patient can then place their arms in the arm restraining element in a much more comfortable position than in the prior art. Furthermore, their elbows stick out less from their trunk, which means that patients whose underarms have been operated on experience reduced discomfort and possible collisions with the gantries of imaging machines (CT, PET, MR) or treatment machines (linear accelerators) are also reduced.

In principle, the movement of the restraining element between the retracted and extended positions can be performed in any way known in the art. For example, it would be possible to design a rotating mechanism, or with a combination of rotational and translational movements, capable of transitioning from the retracted position to the extended position according to a circular line of movement. However, in a particularly preferred embodiment of the invention the restraining element is sliding between the retracted position and the extended position. More specifically, in another more preferred embodiment of the invention the restraining element is sliding between the retracted position and the extended position parallel to the longitudinal direction of the inclined plate.

More preferably, the arm restraining means comprise a support which is arranged at the upper end of the inclined plate projecting vertically above same and to which there is longitudinally coupled in a sliding manner the restraining element. The sliding element can have several intermediate positions between the extended position and the retracted position. For example, the coupling between the support and the restraining element can be made through a rack rod such that the arm restraining element can be fixed at any intermediate point between the extended and retracted positions.

The inventor has also discovered that the patient arm fixing means in the devices of the prior art constitute another reason for the lack of reproducibility in immobilisation results. In fact, the arm restraining means commonly used consist simply of patient arm supporting elements or gripping elements. When the patient is immobilised in the same position for a long time, they become tired because they must exert muscular effort to keep gripping on the gripping element or to keep their arms supported on the supporting elements without falling. Because of this tiredness, they tend to move their arms, and this constitutes a problem.

Preferably, in the present invention the restraining element has arm fixing means actively restraining the arms of the patient. In this context, "actively" restraining the arms of the patient means that the fixing means are configured such that it is possible to fix the arms of the patient, for example their wrists or hands, to said fixing means. This allows the patient to simply let their arms go limp if they are tired of keeping their arms in a specific position, with their arms being restrained by the fixing means without changing the flexion of the arm. The feeling of tiredness, and therefore also the patient's need to move their arms, are thereby prevented. As a result, a further improvement in the reproducibility of immobilisation is achieved. These fixing means can adopt any shape, although according to another more preferred embodiment of the present invention the fixing means comprise wrist straps configured for immobilising the wrists of the patient.

According to yet another preferred embodiment of the invention, the immobilisation device further comprises stereotactic marks arranged on the outer face of the side contention walls. Additionally, the immobilisation device of the present invention may comprise a stereotactic arch that can be coupled to the inclined plate. These stereotactic marks and arches provide references to the medical personnel for assuring the reproducibility of patient immobilisation. In these embodiments, the device therefore becomes a stereotactic immobilisation device according to the definition provided herein above.

According to another embodiment of the present invention, the side plates and/or the stereotactic arch have a series of holes which allow seeing the stereotactic coordinates. This allows assuring the coincidence of lasers tracing the orthogonal planes of the imaging and treatment machines on the device, and allowing these very references to be seen in radiological images. This configuration allows linking the external anatomy of the patient, the equipment, and their internal organs with the stereotactic coordinates established through the holes at all times.

According to another more preferred embodiment of the invention, the immobilisation device comprises first fixing means located in the inclined plate for the antenna of magnetic resonance equipment. These first means can be formed by an antenna-supporting holder that projects through a side area of the inclined plate and are provided with elements for supporting the antenna, thereby preventing the patient from having to hold it.

According to another embodiment of the present invention, the device further comprises second fixing means in the inclined plate for a thermoplastic immobilisation blanket of the patient. Said thermoplastic blanket allows controlling the respiratory movements of the patient to increase the precision of the radiotherapy treatment. These second fixing means can be configured, for example, as a groove or openings arranged in a side area of the inclined plate.

According to an alternative aspect of the present invention, the device can be formed only by the inclined plate or only by the lower rotatable platform. In other words, the inclined plate and the lower rotatable platform can be used independently of one another.

In other words, the inclined plate may be used without the described lower rotatable platform coupled to its lower end. In this case, the patient would be placed on the inclined plate in a manner similar to the conventional manner. The inclined plate of this particular embodiment keeps all the auxiliary elements herein described, such as the V-shaped sub-plates, the contention walls, the central longitudinal cavity, the arm restraining means, the stereotactic marks, the stereotactic arch, and the fixing means for the antenna of magnetic resonance equipment, or a thermoplastic immobilisation blanket.

Similarly, the lower rotatable platform may be used without the described inclined plate coupled thereto. In this case, the lower rotatable platform would be used by means of coupling same to an inclined plate of the type that is conventionally known, providing said conventional inclined plate with advantages inherent to the rotatable platform of the invention. The lower rotatable platform of this particular embodiment keeps all the auxiliary elements herein described, such as the vertical protuberance and the vertical side plates.

### BRIEF DESCRIPTION OF THE DRAWINGS

Figure 1 shows a perspective view of a first example of an immobilisation device according to the prior art.
Figure 2 shows a perspective view of a second example of an immobilisation device according to the prior art.
Figure 3 shows a perspective view of an immobilisation device according to the present invention.
Figures 4a, 4b, and 4c, respectively, show a perspective view, a profile view, and a plan view of a lower rotatable platform according to the present invention.
Figures 5a and 5b show respective side views of an inclined plate provided with arm restraining means according to the present invention (the lower rotatable platform is not shown for the sake of simplicity).
Figures 6a to 6f show different steps representative of a method of using the device according to the present invention.

### PREFERRED EMBODIMENT OF THE INVENTION

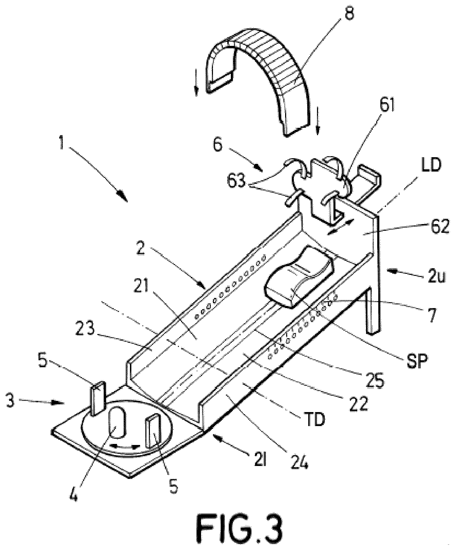

Figure 3 shows a general perspective view of the immobilisation device (1) according to the present invention. This device (1) is fundamentally formed by two elements: a lower platform (3) and an inclined plate (2). The longitudinal and transverse directions are depicted by means of respective lines referred to as (LD) and (TD), respectively.

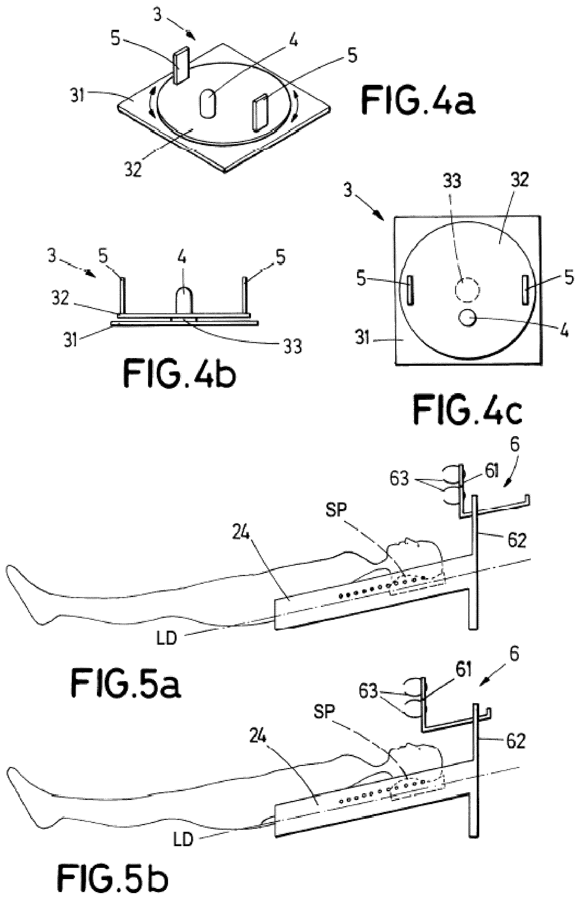

In relation to the platforms used in the devices of the prior art shown in Figures 1 and 2, the lower platform (3) has the particularity that it is rotatable. Specifically, as can be seen in greater detail in Figures 4a to 4c, this platform (3) is formed by a base (31) which, in this example, has a square shape, although the base (31) can in principle have any shape as long as it provides a firm support for supporting the patient's weight. Specifically, the base (31) is rotatably coupled to a rotatable plate (32) on which the patient will sit. In this example, the rotatable plate (32) has a round shape, although this is also not essential, and the base may adopt other shapes, if necessary. The rotatable plate (32) is coupled to the base (31) through a rotary attachment (33). In terms of size, the rotatable plate (32) of the platform (3) will have a suitable size for a patient to sit thereon.

Figure 3 also shows a protuberance (4) intended for being positioned between the legs of the patient, supported on their perineum to prevent them from sliding down according to the longitudinal direction of the inclined plate (2). This protuberance (4), which is shown in greater detail in Figures 4a to 4c, has a shape that is devoid of sharp edges to be comfortably supported on the inner thigh of the patient. Specifically, in this example it has a cylindrical shape with a semi-spherical upper end. The diameter of this protuberance (4) is configured so that it fits comfortably between the legs of the patient, and can be between 8 cm and 15 cm, for example. Its height will also be sufficient to prevent the patient from being able to slide over it, and can be between 10 cm and 20 cm, for example. With respect to its position, it is important to highlight that it is not essential for the protuberance to be arranged exactly in the centre of the rotatable plate (32). However, when the rotatable plate (32) is in the usage orientation, it is important for the protuberance (4) to be transversely centred and arranged at a certain distance from the edge of the base (31) which is coupled to the lower end (21) of the inclined plate which allows comfortably accommodating the buttocks of the patient.

Figure 3 also shows a pair of parallel vertical plates (5) facing one another and located in the peripheral area of the circumference of the rotatable plate (32). These plates (5) are separated by an approximate distance of 40 cm for constraining the hips of the patient and preventing them from moving. The dimensions of the plates (5) are selected so that they constrain the hips of the patient without damaging them with their sharp edges, either the sharp side edges or the sharp upper edge. In this example, the plates (5) have a length of 10 cm and a height of 15 cm. Furthermore, it is not essential for the plates (5) to be flat, where they can have a small curvature towards the inside of the rotatable plate (32) to adapt to the hips of the patient in a more ergonomic manner. Moreover, with respect to their position, it is not strictly necessary for the plates to be arranged in diametrically opposing positions. It also not essential for both plates (5) to be aligned with the protuberance (4), as it can be slightly shifted in the direction perpendicular to said line joining the plates (5), where required, for maximum patient comfort.

The inclined plate (2) has an essentially rectangular shape and is arranged according to an inclination in a longitudinal direction coinciding with the direction of its long side. The short lower side, located at its lower end (21), is coupled to a side of the rotatable platform (3) through any means suitable to that end, such as press fit, tongue and groove joints, or others, for example. As discussed above, these coupling means can be of variable length. The short upper side, located at its upper end (2u), is supported on rigid legs which keep the angle of inclination fixed and unchanging at a value of about 10°. An important particularity of the described inclined plate (2) is that the surface on which the patient resist is not strictly flat, but rather is formed by two V-shaped sub-plates (21, 22) having a small inclination towards the centre of the plate (2). Each of the sub-plates (21) can have an essentially elongated rectangular shape the ends of which are fixed to a respective supporting elements located at the lower and upper ends (21, 2u) of said plate (2). This inclination helps to immobilise the patient even more while using the device (1) of the invention.

The plate (2) furthermore has essentially vertical contention walls (23, 24) located along its side edges. In this example, there are two straight vertical walls (23, 24) that span both side edges of the plate (2) entirely and have an approximate height in relation to the surface of the plate (2) of about 9 cm. The patient is thus fitted between these two walls (23, 24) with hardly any margin for movement. The walls (23, 24) furthermore have on their outer face graduated stereotactic marks (7) which allow a precise positioning of the patient. They also have a series of openings and fixing elements intended for the coupling of various elements used during radiotherapy treatment, such as the antenna for magnetic resonance equipment, a thermoplastic immobilisation blanket, or the described stereotactic arch (8), for example.

Figure 3 also shows how the plate (2) has a central longitudinal cavity (25) separating both sub-plates (21, 22). This central longitudinal cavity (25) is intended for housing the spinous process of the patient, which results not only in greater comfort but also in increased precision during positioning. In this example, the width of the central longitudinal cavity (25) is 6 cm.

The device (1) of the invention further comprises fixing means (6) for fixing the arms of the patient. These fixing means (6) comprise a restraining element (61) movable between a retracted position and an extended position to which the arms of the patient are fixed. In this example, the restraining element (61) adopts the shape of an essentially flat plate to which constraining means in the form of wrist straps (63) are fixed. These wrist straps (63) constitute a significant advantage in relation to the fixing elements used in the immobilisation devices known today, as they allow firmly constraining the wrists of the patient and the patient therefore does not have to make any effort to keep their arms still and away from the treatment area.

The fixing means (6) further comprise a supporting plate (62) located at the upper end (2u) of the plate. This supporting plate (62) has an opening through which a rod slides at the end of which there is arranged the fixing element (61). In that sense, as shown in Figure 5a, when the fixing element (61) is in the retracted position, it is arranged adjacent to the supporting plate (62), in a position longitudinally above the supporting position (SP) for supporting the patient's head. In this supporting position (SP), a headrest of the type conventionally used in immobilisation devices of this type has been shown. Therefore, when the fixing element (61) is in the retracted position the patient can lean back normally until their head is placed in said supporting position (SP). Figure 5b shows the fixing element (61) in an extended position. As can be seen, in the extended position the fixing element (61) is located according to the longitudinal direction at the height of the supporting position (SP) for supporting the patient's head. Furthermore, the lower end of the fixing element (61) is sufficiently separated from the inclined plate (2) so as to leave enough space for the patient's head. In this example, the distance is 26 cm. As a result of this configuration, in this device the arms of the patient are immobilised in a much more comfortable position than in earlier devices, for example approximately in front of their eyes or forehead.

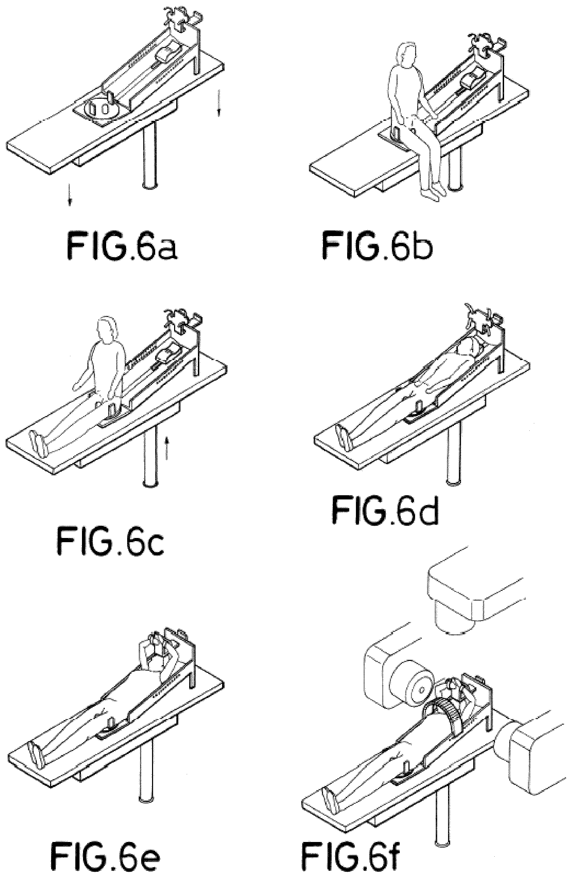

Figures 6a to 6f show the different steps of a process of immobilising a patient using the device of the present invention. Figure 6a shows the immobilisation device (1) of the invention placed on the stretcher of a linear accelerator. Figure 6b shows a first step in which the patient has sat on the lower platform (3). To that end, the stretcher has previously moved down from its initial height and the rotatable plate (32) of the platform (3) has furthermore been placed in the initial orientation. In this example, the initial orientation corresponds to the one depicted in Figures 6a and 6b, in which the plates (5) are aligned according to the longitudinal direction. With this orientation of the rotatable plate (32), the patient only has to lower themselves until they are seated such that the protuberance (4) is arranged between their legs and their hips are fitted between the plates (5). Next, Figure 6c shows the subsequent step in which the medical personnel turns the rotatable plate (32) 90° from its initial position to its usage position. In this example, the usage position is the one depicted from Figures 6c onwards, in which the line joining the plates (5) is perpendicular to the longitudinal direction of the device (1). To enable performing this 90° turn of the rotatable plate (32) with the patient seated thereon, the legs of the patient are lifted. This results in the patient being aligned according to the longitudinal direction of the device. Next, as seen in Figure 6d, the patient simply leans back until they rest on the inclined plate (2). The spinous process of the patient is aligned in the central longitudinal cavity (25), and the rest of the back is supported on the sub-plates (21, 22) inclined towards the central longitudinal line of the plate (2). The shoulders of the patient are fitted against the side contention walls (23, 24) of the inclined plate (2). Finally, at the end of this step the patient's entire trunk is constrained by the different elements of the inclined plate (2) and their pelvis and perineal area are also immobilised by the corresponding elements of the lower rotatable platform (3). Figure 6e shows the subsequent step consisting of the immobilisation of the arms of the patient. To that end, the arm fixing element (61) slides from the retracted position shown in Figure 6d to the extended position shown in Figure 6e. The arms of the patient are fixed to the arm fixing element (61) by means of wrist straps (63). This results in the arms of the patient being fixed approximately in front of their eyes or forehead, and furthermore the patient can allow their arms "to go limp" such that they hang from the wrist straps, thereby avoiding the exertion of any effort will may tire the patient out. The patient is already completely immobilised and ready to start treatment. In a last step, Figure 6f shows how the stereotactic arch (8) is fixed to the inclined plate (2).

## Claims

1. An immobilisation device (1) for radiotherapy, comprising:
- an inclined plate (2) for supporting the back of a patient, wherein said plate (2) is inclined in a longitudinal direction and has a lower end (21) and an upper end (2u); and
- a lower platform (3) for supporting the buttocks of the patient, wherein said lower platform (3) is configured for being arranged adjacent to the lower end (2a) of the inclined plate (2),
wherein the immobilisation device (1) is **characterised in that** the lower platform (3) is rotatable to make it easier to place the patient on the device (1), and the lower platform (3) comprises a vertically projecting protuberance (4) which is transversely centred in relation to the inclined plate (2) when it is in a usage orientation, wherein the protuberance (4) is configured such that it can be introduced between the legs of the patient.

2. The device (1) according to claim 1, wherein the inclined plate (2) is formed by two V-shaped side sub-plates (21, 22) which are additionally inclined in a transverse direction so as to prevent the patient from being able to turn.

3. The device (1) according to any of claims 1 or 2, wherein the lower platform (3) comprises lateral areas according to the usage orientation a pair of vertically projecting plates (5) which are configured for compressing the hips of the patient.

4. The device (1) according to any of claims 1 to 3, wherein the inclined plate (2) further comprises two side contention walls (23, 24) projecting essentially in a vertical manner for closely housing the patient.

5. The device (1) according to any of claim 1, wherein the inclined plate (2) is formed by two V-shaped side sub-plates (21, 22) which are additionally inclined in a transverse direction so as to prevent the patient from being able to turn; wherein the lower platform (3) comprises lateral areas according to the usage orientation a pair of vertically projecting plates (5) which are configured for compressing the hips of the patient; and wherein the inclined plate (2) further comprises two side contention walls (23, 24) projecting essentially in a vertical manner for closely housing the patient.

6. The device (1) according to any of the preceding claims, wherein the inclined plate (2) comprises a central longitudinal cavity (25) to make it easier to position the spinous process of the patient.

7. The device (1) according to any of the preceding claims, wherein the inclined plate (2) comprises at its upper end (2u) arm restraining means (6) comprising a restraining element (61), wherein said restraining element (61) is movable between a retracted position located longitudinally above a supporting position (SP) for supporting the patient's head, and an extended position located longitudinally at the height of the supporting position (SP) for supporting the patient's head, and wherein in the extended position the lower end of the restraining element (61) is perpendicularly separated from the inclined plate (2) by a distance that is enough for accommodating the patient's head such that it is supported on said supporting position (SP).

8. The device (1) according to claim 7, wherein the distance between the inclined plate (2) and the lower end of the restraining element (61) when said restraining element (61) is in the extended position is between 20 cm and 30 cm.

9. The device (1) according to any of claims 7 to 8, wherein the restraining element (61) is sliding between the retracted position and the extended position.

10. The device (1) according to claim 9, wherein the arm restraining means (6) comprise a support (62) arranged at the upper end (2u) of the inclined plate projecting vertically above the plate to which there is coupled in a sliding manner in the longitudinal direction the restraining element (61) and, wherein the restraining element (61) has arm fixing means (63) actively restraining the arms of the patient.

11. The device (1) according to any of the preceding claims, further comprising graduated stereotactic marks (7) arranged on the outer face of the side contention walls (23, 24).

12. The device (1) according to any of the preceding claims, further comprising a stereotactic arch (8) that can be coupled to the inclined plate (2).

13. The device (1) according to any of claims 10 to 11, wherein the side contention walls (23, 24) and/or the stereotactic arch (8) have a series of holes which allow seeing the stereotactic marks.

14. The device (1) according to any of the preceding claims, further comprising first fixing means located in the inclined plate (2) for supporting the antenna of magnetic resonance equipment.

15. The device (1) according to any of the preceding claims, further comprising second fixing means located in the inclined plate (2) for a thermoplastic immobilisation blanket of the patient.

## Patentansprüche

1. Immobilisierungsvorrichtung (1) für die Strahlentherapie, umfassend:
- eine geneigte Platte (2) zur Abstützung des Rückens eines Patienten, wobei die genannte Platte (2) in einer Längsrichtung geneigt ist und ein unteres Ende (2l) und ein oberes Ende (2u) aufweist; und
- eine untere Plattform (3) zur Abstützung des Gesäßes des Patienten, wobei die genannte untere Plattform (3) dazu ausgebildet ist, um dem unteren Ende (2a) der geneigten Platte (2) benachbart angeordnet zu werden,
wobei die Immobilisierungsvorrichtung (1) **dadurch gekennzeichnet ist, dass** die untere Plattform (3) rotierbar ist, um es einfacher zu machen, den Patienten auf der Vorrichtung (1) zu platzieren, und die untere Plattform (3) einen vertikal hervorstehenden Vorsprung (4) umfasst, welcher in Bezug auf die geneigte Platte (2) quer zentriert ist, wenn sie in einer Nutzungsorientierung ist, wobei der Vorsprung (4) derart ausgebildet ist, dass er zwischen den Beinen des Patienten eingeführt werden kann.

2. Vorrichtung (1) nach Anspruch 1, in welcher die geneigte Platte (2) aus zwei V-förmigen seitlichen Teilplatten (21, 22) gebildet ist, welche zusätzlich in einer Querrichtung geneigt sind, um zu verhindern, dass sich der Patient drehen kann.

3. Vorrichtung (1) nach einem der Ansprüche 1 oder 2, in welcher die untere Plattform (3) in Seitenbereichen gemäß der Nutzungsorientierung ein Paar von vertikal hervorstehenden Platten (5) umfasst, welche dazu ausgebildet sind, die Hüften des Patienten zusammenzudrücken.

4. Vorrichtung (1) nach einem derAnsprüche 1 bis 3, in welcherdie geneigte Platte (2) zusätzlich zwei seitliche Sicherheitswände (23, 24) umfasst, welche im Wesentlichen vertikal hervorstehen, um den Patienten eng aufzunehmen.

5. Vorrichtung (1) nach einem des Anspruchs 1, in welcher die geneigte Platte (2) aus zwei V-förmigen seitlichen Teilplatten (21, 22) gebildet ist, welche zusätzlich in einer Querrichtung geneigt sind, um zu verhindern, dass sich der Patient drehen kann; in welcher die untere Plattform (3) in Seitenbereichen gemäß der Nutzungsorientierung ein Paar von vertikal hervorstehenden Platten (5) umfasst, welche dazu ausgebildet sind, die Hüften des Patienten zusammenzudrücken; und in welcher die geneigte Platte (2) zusätzlich zwei seitliche Sicherheitswände (23, 24) umfasst, welche im Wesentlichen vertikal hervorstehen, um den Patienten eng aufzunehmen.

6. Vorrichtung (1) nach einem der vorhergehenden Ansprüche, in welcher die geneigte Platte (2) einen zentralen länglichen Hohlraum (25) umfasst, um es einfacher zu machen, den Dornfortsatz des Patienten zu positionieren.

7. Vorrichtung (1) nach einem der vorhergehenden Ansprüche, in welcher die geneigte Platte (2) an deren oberen Ende (2u) Armrückhaltemittel (6) umfasst, welche ein Rückhalteelement (61) umfassen, wobei das genannte Rückhalteelement (61) zwischen einer eingefahrenen Stellung, welche sich längs über einer Stützstellung (SP) befindet, zur Abstützung des Kopfs des Patienten, und einer ausgefahrenen Stellung, welche sich längs auf der Höhe der Stützstellung (SP) befindet, zur Abstützung des Kopfs des Patienten, beweglich ist, und in welcher in der ausgefahrenen Stellung das untere Ende des Rückhalteelements (61) mit einem Abstand von der geneigten Platte (2) senkrecht beabstandet ist, welcher Abstand ausreichend ist, um den Kopf des Patienten derart unterzubringen, dass er auf der genannten Stützstellung (SP) abgestützt ist.

8. Vorrichtung (1) nach Anspruch 7, in welcher der Abstand zwischen der geneigten Platte (2) und dem unteren Ende des Rückhalteelements (61), wenn das genannte Rückhalteelement (61) in der ausgefahrenen Stellung ist, zwischen 20 cm und 30 cm liegt.

9. Vorrichtung (1) nach einem der Ansprüche 7 bis 8, in welcher das Rückhalteelement (61) zwischen der eingefahrenen Stellung und der ausgefahrenen Stellung gleitet.

10. Vorrichtung (1) nach Anspruch 9, in welcher die Armrückhaltemittel (6) eine Stütze (62) umfassen, welche im oberen Ende (2u) der geneigten Platte angeordnet ist, welches vertikal über der Platte hervorsteht, mit welchem in der Längsrichtung das Rückhalteelement (61) gleitend gekoppelt ist und, in welcher das Rückhalteelement (61) Armfixierungsmittel (63) aufweist, welche die Arme des Patienten aktiv zurückhalten.

11. Vorrichtung (1) nach einem der vorhergehenden Ansprüche, zusätzlich umfassend abgestufte stereotaktische Marken (7), welche auf der Außenfläche der seitlichen Sicherheitswände (23, 24) angeordnet sind.

12. Vorrichtung (1) nach einem der vorhergehenden Ansprüche, zusätzlich umfassend einen stereotaktischen Bogen (8), welcher mit der geneigten Platte (2) gekoppelt werden kann.

13. Vorrichtung (1) nach einem der Ansprüche 10 bis 11, in welcher die seitlichen Sicherheitswände (23, 24) und/oder der stereotaktische Bogen (8) eine Reihe von Löchern aufweisen, welche es erlauben, die stereotaktischen Marken zu sehen.

14. Vorrichtung (1) nach einem der vorhergehenden Ansprüche, zusätzlich umfassend erste Fixierungsmittel, welche sich in der geneigten Platte (2) befinden, um die Antenne des Magnetresonanzgeräts abzustützen.

15. Vorrichtung (1) nach einem der vorhergehenden Ansprüche, zusätzlich umfassend zweite Fixierungsmittel, welche sich in der geneigten Platte (2) befinden, für eine thermoplastische Immobilisierungsdecke des Patienten.

## Revendications

1. Dispositif d'immobilisation (1) pour radiothérapie, qui comprend :
- une plaque inclinée (2) pour supporter le dos d'un patient, dans lequel ladite plaque (2) est inclinée dans une direction longitudinale et a une extrémité inférieure (2l) et une extrémité supérieure (2u) ; et
- une plateforme inférieure (3) pour supporter les fesses du patient, dans lequel ladite plateforme inférieure (3) est configurée pour être disposée adjacente à l'extrémité inférieure (2a) de la plaque inclinée (2),
dans lequel le dispositif d'immobilisation (1) est **caractérisé en ce que** la plateforme inférieure (3) peut tourner pour rendre plus facile de placer le patient sur le dispositif (1), et la plateforme inférieure (3) comprend une protubérance en saillie verticalement (4) qui est centrée transversalement par rapport à la plaque inclinée (2) lorsqu'elle est dans une orientation d'utilisation, dans lequel la protubérance (4) est configurée de telle sorte qu'elle peut être introduite entre les jambes du patient.

2. Dispositif (1) selon la revendication 1, dans lequel la plaque inclinée (2) est formée de deux sous-plaques latérales en forme de V (21, 22) qui sont en inclinées de surcroît dans une direction transversale pour empêcher que le patient puisse se tourner.

3. Dispositif (1) selon l'une quelconque des revendications 1 ou 2, dans lequel la plateforme inférieure (3) comprend dans des zones latérales selon l'orientation d'utilisation une paire de plaques en saillie verticalement (5) qui sont configurées pour comprimer les hanches du patient.

4. Dispositif (1) selon l'une quelconque des revendications 1 à 3, dans lequel la plaque inclinée (2) comprend en outre deux parois de contention latérales (23, 24) qui sont en saillie essentiellement d'une manière verticale pour loger étroitement le patient.

5. Dispositif (1) selon l'une quelconque de la revendication 1, dans lequel la plaque inclinée (2) est formée de deux sous-plaques latérales en forme de V (21, 22) qui sont inclinées de surcroît dans une direction transversale pour empêcher que le patient puisse se tourner ; dans lequel la plateforme inférieure (3) comprend dans des zones latérales selon l'orientation d'utilisation une paire de plaques en saillie verticalement (5) qui sont configurées pour comprimer les hanches du patient ; et dans lequel la plaque inclinée (2) comprend en outre deux parois de contention latérales (23, 24) qui sont en saillie essentiellement de manière verticale pour loger étroitement le patient.

6. Dispositif (1) selon l'une quelconque des revendications précédentes, dans lequel la plaque inclinée (2) comprend une cavité longitudinale centrale (25) pour rendre plus facile de positionner l'apophyse épineuse du patient.

7. Dispositif (1) selon l'une quelconque des revendications précédentes, dans lequel la plaque inclinée (2) comprend sur son extrémité supérieure (2u) des moyens de restriction de bras (6) qui comprennent un élément de restriction (61), dans lequel ledit élément de restriction (61) peut se déplacer entre une position rétractée située longitudinalement au-dessus d'une position de support (SP) pour supporter la tête du patient, et une position étendue située longitudinalement à la hauteur de la position de support (SP) pour supporter la tête du patient, et dans lequel, dans la position étendue, l'extrémité inférieure de l'élément de restriction (61) est séparée perpendiculairement de la plaque inclinée (2) par une distance qui est suffisante pour loger la tête du patient de telle manière qu'elle est supportée dans ladite position de support (SP).

8. Dispositif (1) selon la revendication 7, dans lequel la distance entre la plaque inclinée (2) et l'extrémité inférieure de l'élément de restriction (61) lorsque ledit élément de restriction (61) est dans la position étendue est comprise entre 20 cm et 30 cm.

9. Dispositif (1) selon l'une quelconque des revendications 7 à 8, dans lequel l'élément de restriction (61) coulisse entre la position rétractée et la position étendue.

10. Dispositif (1) selon la revendication 9, dans lequel les moyens de restriction de bras (6) comprennent un support (62) disposé sur l'extrémité supérieure (2u) de la plaque inclinée qui est en saillie verticalement au-dessus de la plaque à laquelle l'élément de restriction (61) est accouplé de manière coulissante dans la direction longitudinale et dans lequel l'élément de restriction (61) a des moyens de fixation de bras (63) qui retiennent activement les bras du patient.

11. Dispositif (1) selon l'une quelconque des revendications précédentes, qui comprend en outre des marques stéréotaxiques graduées (7) disposées sur la face externe des parois de contention latérales (23, 24).

12. Dispositif (1) selon l'une quelconque des revendications précédentes, qui comprend en outre un arc stéréotaxique (8) qui peut être accouplé à la plaque inclinée (2).

13. Dispositif (1) selon l'une quelconque des revendications 10 à 11, dans lequel les parois de contention latérales (23, 24) et/ou l'arc stéréotaxique (8) ont une série d'orifices qui permettent de voir les marques stéréotaxiques.

14. Dispositif (1) selon l'une quelconque des revendications précédentes, qui comprend en outre de premiers moyens de fixation situés sur la plaque inclinée (2) pour supporter l'antenne d'un équipement de résonnance magnétique.

15. Dispositif (1) selon l'une quelconque des revendications précédentes, qui comprend en outre de seconds moyens de fixation sur la plaque inclinée (2) pour une couverture d'immobilisation thermoplastique du patient.
